# EUROPÄISCHE PATENTANMELDUNG

(11) **EP 0 647 627 A1**
(43) Veröffentlichungstag der Anmeldung: **12.04.1995**
(21) Anmeldenummer: 94115091.4
(22) Anmeldetag: 24.09.1994
(51) Int. Cl.: C07D 213/64, C07D 401/10, A61K 31/44

(54) **1,2-Dihydro-2-oxo-3-Methylsulfonylaminomethyl-Pyridine als Angiotensin II Antagonisten**

(30) Priorität: 08.10.1993 DE 4334308
(71) Anmelder: MERCK PATENT GmbH, D-64293 Darmstadt (DE)
(72) Erfinder: Osswald, Mathias, Dr., D-64673 Zwingenberg (DE); Mederski, Werner, Dr., D-64390 Erzhausen (DE); Dorsch, Dieter, Dr., D-64372 Ober-Ramstadt (DE); Beier, Norbert, Dr., D-64354 Reinheim 5 (DE); Schelling, Pierre, Prof. Dr., D-64367 Mühltal (DE); Minck, Klaus-Otto, Dr., D-64372 Ober-Ramstadt (DE); Lues, Ingeborg, Dr., D-64297 Darmstadt (DE)

(57) **Zusammenfassung**

Neue 1,2-Dihydro-2-oxo-3-methylsulfonylaminomethyl-pyridine der Formel I 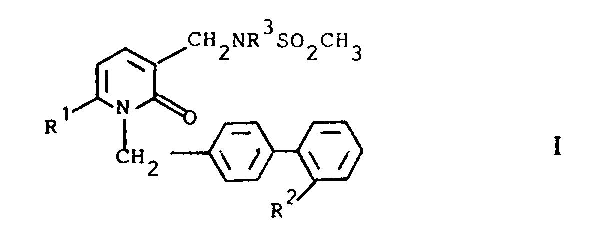
worin
- R¹: Alkyl mit 1-5 C-Atomen,
- R²: CN oder 1H-5-Tetrazolyl und
- R³: Alkyl mit 1-5 C-Atomen oder Cyclopropylmethyl bedeuten,
sowie deren Salze zeigen angiotensin II-antagonistische Eigenschaften und können zur Behandlung von Hypertension, Aldosteronismus und Herzinsuffizienz verwendet werden.

## Beschreibung

Die Erfindung betrifft neue 1,2-Dihydro-2-oxo-3-methylsulfonylaminomethyl-pyridine der Formel I 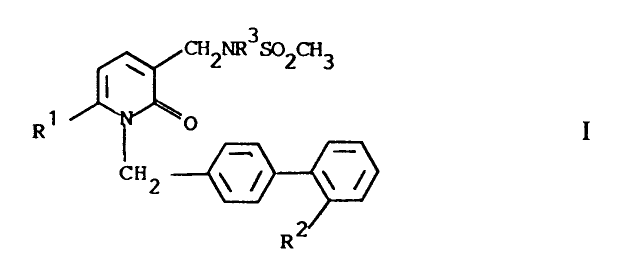
worin
- R¹: Alkyl mit 1-5 C-Atomen,
- R²: CN oder 1H-5-Tetrazolyl und
- R³: Alkyl mit 1-5 C-Atomen oder Cyclopropylmethyl bedeuten,
sowie ihre Salze.

Ähnliche Verbindungen sind aus EP 0 530 702 A1 bekannt. In dieser Druckschrift ist eine Formel "I" angegeben 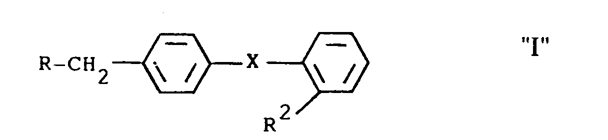
worin R 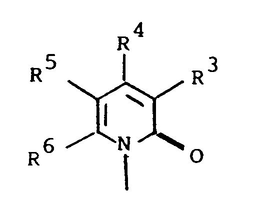
bedeutet und
- R¹, R⁴ und R⁵: jeweils auch H,
- R²: auch CN oder 5-Tetrazolyl,
- R³: auch CH₂NR¹⁰R¹¹,
- R⁶ und R¹⁰: jeweils auch Alkyl und
- R¹¹: auch SO₂R⁷ (worin R⁷ auch Alkyl sein kann) bedeuten sowie
- X: auch fehlen kann.

An keiner Stelle dieser Druckschrift werden jedoch Verbindungen der vorliegenden Formel I erwähnt oder nahegelegt, auch nicht einzelne unter diese Formel fallende Verbindungen. Bei der vorliegenden Erfindung handelt es sich daher im Hinblick auf diese Druckschrift um eine Auswahlerfindung, soweit Verbindungen betroffen sind, in denen R¹ und R³ jeweils Alkyl mit 1-5 C-Atomen bedeuten.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre Salze bei guter Verträglichkeit sehr wertvolle pharmakologische Eigenschaften besitzen. Insbesondere zeigen sie angiotensin II-antagonistische Eigenschaften und können daher als Arzneimittelwirkstoffe in der Human- und Veterinärmedizin eingesetzt werden, insbesondere zur Prophylaxe und/oder Therapie von Herz-, Weislauf- und Gefäßkrankheiten, vor allem zur Behandlung der angiotensin II-abhängigen Hypertension, des Aldosteronismus, der Herzinsuffizienz und des erhöhten Augeninnendrucks sowie von Störungen des Zentralnervensystems, ferner von Hypertrophie und Hyperplasie der Blutgefäße und des Herzens, Angina pectoris, Herzinfarkt, Schlaganfall, Restenosen und Angioplastie nach Bypass-Operationen, von ischämischen peripheren Durchblutungsstörungen, Arteriosklerose, Glaukomen, macularer Degeneration, Hyperurikämie, Nierenfunktionsstörungen, z.B. Nierenversagen, Nephropathia diabetica, Retinopathia diabetica, Psoriasis, von gastointestinalen Erkrankungen, Blasenerkrankungen, Lungenödemen, chronischer Bronchitis, angiotensin II-vermittelten Störungen in weiblichen Fortpflanzungsorganen, Wahrnehmungsstörungen, z.B. Demenz, Amnesie, Gedächtnisfunktionsstörungen, Angstzuständen, Depression, Epilepsie, des Parkinson-Syndroms und/oder der Bulume.

Diese Wirkungen können nach üblichen in vitro- oder in vivo-Methoden ermittelt werden, wie sie z.B. in der US-PS 4 880 804, der US-PS 5 036 048 und der WO 91/14367 beschrieben sind, ferner von A.T. Chiu et al., J. Pharmacol. Exp. Therap. 250, 867-874 (1989), und von P.C. Wong et al., ibid. 252, 719-725 (1990; in vivo, an Ratten).

So zeigten die K-Salze von 1,2-Dihydro-1-(2'-(1H-5-tetrazolyl)-biphenylyl-4-methyl)-2-oxo-3-N-methyl- bzw. -3-N-isopropyl-N-methylsulfonylaminomethyl-6-butyl-pyridin ("Iy" bzw. "Iz") an der Rattennebenniere (Versuchsmethodik vgl. A.T.Chiu et al., l.c.) IC 50-Werte von 1,15 bzw. 1,9 nM.l-¹, wahrend das bekannte 1,2-Dihydro-1-(2'-(1H-5-tetrazolyl)biphenylyl-4-methyl)-2-oxo-3-methylsulfonylaminomethyl-6-butyl-pyridin (vgl. EP 0 530 702 A1, Beispiel 22) im gleichen Test einen IC 50-Wert von 4,0 nM · l⁻¹ aufwies.

Gegenstand der Erfindung sind die Verbindungen der Formel I und ihre Salze sowie ein Verfahren zur Herstellung dieser Verbindungen sowie ihrer Salze, dadurch gekennzeichnet, daß man
a) eine Verbindung der Formel II 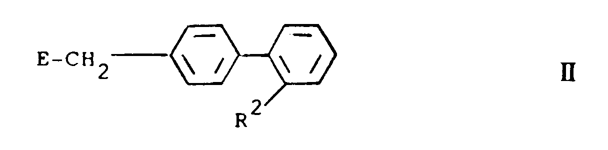 worin
   - E: Cl, Br, I oder eine freie oder reaktionsfähig funktionell abgewandelte OH-Gruppe bedeutet und
   - R²: die bei Formel I angegebene Bedeutung hat,
   mit einer Verbindung der Formel III 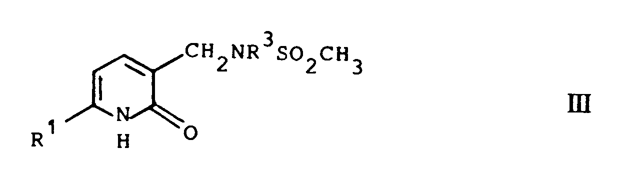 worin
   R¹ und R³ die bei Formel I angegebenen Bedeutungen haben, oder einem reaktionsfähigen Derivat dieser Verbindung umsetzt, oder daß man
b) eine Verbindung der Formel I aus einem ihrer funktionellen Derivate durch Behandeln mit einem solvolysierenden oder hydrogenolysierenden Mittel in Freiheit setzt, oder daß man
c) eine Verbindung der Formel IV 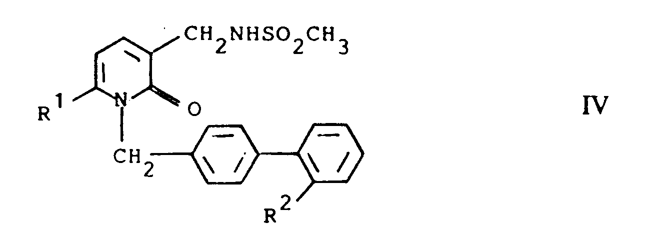 worin
   R¹ und R² die bei Formel I angegebenen Bedeutungen haben, oder ein reaktionsfähiges Derivat dieser Verbindung
   mit einer Verbindung der Formel V

   E-R³ V

   worin
   E und R³ die bei den Formeln II bzw. I angegebenen Bedeutungen haben,
   umsetzt
und/oder daß man in einer Verbindung der Formel I, worin R² CN bedeutet, diesen Rest CN in eine 1H-5-Tetrazolylgruppe umwandelt und/oder eine Verbindung der Formel I, worin R² 1H-5-Tetrazolyl bedeutet, durch Behandeln mit einer Base in eines ihrer Salze umwandelt.

Vor- und nachstehend haben die Reste R¹, R², R³ und E die bei den Formeln I und II angegebenen Bedeutungen, falls nicht ausdrücklich etwas anderes angegeben ist.

In den vorstehenden Formeln hat Alkyl 1-5, vorzugsweise 1, 2, 3, oder 4 C-Atome, und bedeutet vorzugsweise Methyl, Ethyl, Propyl, Isopropyl, Butyl, Isobutyl, sek.-Butyl, oder tert.-Butyl, ferner auch Pentyl, 1-, 2- oder 3-Methylbutyl, 1,1-, 1,2-, 2,2-Dimethylpropyl oder 1-Ethylpropyl.

Der Rest R¹ ist vorzugsweise Butyl, ferner bevorzugt Ethyl oder Propyl.

Der Rest R² ist vorzugsweise 1H-5-Tetrazolyl. Die Nitrile der Formel I (R² = CN) sind in erster Linie als Zwischenprodukte zur Herstellung der Tetrazole (I, R² = 1H-5-Tetrazolyl) wichtig, sind aber selbst auch pharmakologisch wirksam.

Der Rest R³ ist vorzugsweise Methyl, Ethyl, Isopropyl oder Cyclopropylmethyl.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart; insbesondere aber US-PS 4 880 804) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.

Die Verbindungen der Formel I können erhalten werden, indem man Verbindungen der Formel II mit Verbindungen der Formel III oder mit deren reaktionsfähigen Derivaten, z.B. deren Alkalimetallsalzen, umsetzt.

In den Verbindungen der Formel II bedeutet E vorzugsweise Cl, Br, I oder eine reaktionsfähig funktionell abgewandelte OH-Gruppe wie Alkylsulfonyloxy mit 1-6 C-Atomen (bevorzugt Methylsulfonyloxy) oder Arylsulfonyloxy mit 6-10 C-Atomen (bevorzugt Phenyl- oder p-Tolylsulfonyloxy).

Die Umsetzung von II mit III erfolgt zweckmäßig, indem man zunächst III durch Behandeln mit einer Base in ein Salz umwandelt, z.B. mit einem Alkalimetallalkoholat wie CH₃ONa in einem Alkohol die CH₃OH oder mit Cäsiumcarbonat, NaH oder K-tert.-Butylat in Dimethylformamid (DMF), und dieses dann in einem inerten Lösungsmittel, z.B. einem Amid wie DMF oder Dimethylacetamid oder einem Sulfoxid wie Dimethylsulfoxid (DMSO), mit II umsetzt, zweckmäßig bei Temperaturen zwischen -20 und 100°, vorzugsweise zwischen 10 und 30°.

Die Ausgangsstoffe der Formeln II und III sind teilweise bekannt. Falls sie nicht bekannt sind, können sie nach bekannten Methoden in Analogie zu bekannten Stoffen hergestellt werden. Verbindungen der Formel II sind weitgehend bekannt (vgl. z.B. EP-A2-400974). Verbindungen der Formel III sind beispielsweise erhältlich durch Reduktion von 1,2-Dihydro-2-oxo-3-cyan-6-R¹-pyridinen zu 1,2-Dihydro-2-oxo-3-formyl-6-R¹-pyridinen, reduktive Aminierung mit Aminen der Formel R³NH₂ zu 1,2-Dihydro-2-oxo-3-(R₃NHCH₂)-6-R¹-pyridinen und Acylierung mit CH₃SO₂Cl.

Man kann ferner eine Verbindung der Formel I aus einem ihrer funktionellen Derivate durch Behandeln mit einem solvolysierenden (z.B. hydrolysierenden) oder hydrogenolysierenden Mittel in Freiheit setzen.

So ist es möglich, nach einer der angegebenen Methoden eine Verbindung herzustellen, die der Formel I entspricht, aber an Stelle einer Tetrazolylgruppe eine funktionell abgewandelte (durch eine Schutzgruppe geschützte) Tetrazolylgruppe enthält. Als Schutzgruppen eignen sich beispielsweise: Triphenylmethyl, abspaltbar mit HCOOH oder HCl in einem inerten Lösungsmittel oder Lösungsmittelgemisch, z.B. Methanol oder Ether/Dichlormethan/Methanol; 2-Cyanethyl, abspaltbar mit NaOH in Wasser/Tetrahydrofuran (THF); p-Nitrobenzyl, abspaltbar mit H₂/Raney-Nickel in Ethanol (vgl. EP-A2-0 291 969).

Eine Verbindung der Formel I ist außerdem erhältlich, indem man ein Sulfonamid der Formel IV (vgl. EP 0 530 702 A1) oder eines seiner reaktionsfähigen Derivate (z.B. seiner Alkalimetallsalze, bevorzugt der Na- oder K-Salze) mit einer Verbindung der Formel V, vorzugsweise einem Alkylhalogenid, umsetzt. Zweckmäßigerweise arbeitet man dabei unter Bedingungen analog zu der Umsetzung von II mit III.

Umsetzung von Nitrilen der Formel I (R² = CN) mit Derivaten der Stickstoffwasserstoffsäure führt zu Tetrazolen der Formel I (R² = 1H-5-Tetrazolyl). Bevorzugt verwendet man Trialkylzinnazide wie Trimethylzinnazid in einem inerten Lösungsmittel, z.B. einem aromatischen Kohlenwasserstoff wie Toluol bei Temperaturen zwischen 20 und 150°, vorzugsweise zwischen 80 und 140°, oder Natriumazid in N-Methylpyrrolidon bei Temperaturen zwischen etwa 100 und 200°.

Verbindungen der Formel I, die Tetrazolylgruppen enthalten können mit Basen (z.B. Natrium- oder Kaliumhydroxid oder -carbonat) in die entsprechenden Metall-, insbesondere Alkalimetall- oder Erdalkalimetall-, oder in die entsprechenden Ammoniumsalze umgewandelt werden. Die Kaliumsalze der Tetrazolylderivate sind besonders bevorzugt.

Die neuen Verbindungen der Formel I und ihre physiologisch unbedenklichen Salze können zur Herstellung pharmazeutischer Präparate verwendet werden, indem man sie zusammen mit mindestens einem Träger- oder Hilfsstoff und, falls erwünscht, zusammen mit einem oder mehreren weiteren Wirkstoff(en) in eine geeignete Dosierungsform bringt. Die so erhaltenen Zubereitungen können als Arzneimittel in der Human- oder Veterinärmedizin eingesetzt werden. Als Trägersubstanzen kommen organische oder anorganische Stoffe in Frage, die sich für die enterale (z.B. orale oder rektale) oder parenterale Applikation oder für eine Applikation in Form eines Inhalations-Sprays eignen und mit den neuen Verbindungen nicht reagieren, beispielsweise Wasser, pflanzliche Öle, Benzylalkohole, Polyethylenglykole, Glycerintriacetat und andere Fettsäureglyceride, Gelatine, Sojalecithin, Kohlenhydrate wie Lactose oder Stärke, Magnesiumstearat, Talk, Cellulose. Zur oralen Anwendung dienen insbesondere Tabletten, Dragees, Kapseln, Sirupe, Säfte oder Tropfen; von Interesse sind speziell Lacktabletten und Kapseln mit magensaftresistenten Überzügen bzw. Kapselhüllen. Zur rektalen Anwendung dienen Suppositorien, zur parenteralen Applikation Lösungen, vorzugsweise ölige oder wässerige Lösungen, ferner Suspensionen, Emulsionen oder Implantate. Für die Applikation als Inhalations-Spray können Sprays verwendet werden, die den Wirkstoff entweder gelöst oder suspendiert in einem Treibgasgemisch (z.B. Kohlenwasserstoffen) enthalten. Zweckmäßig verwendet man den Wirkstoff dabei in mikronisierter Form, wobei ein oder mehrere zusätzliche physiologisch verträgliche Lösungsmittel zugegen sein können, z.B. Ethanol. Inhalationslösungen können mit Hilfe üblicher Inhalatoren verabfolgt werden. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Konservierungs-, Stabilisierungs- und/oder Netzmittel, Emulgatoren, Salze zur Beeinflussung des osmotischen Druckes, Puffersubstanzen, Farb- und/oder Aromastoffe enthalten. Sie können, falls erwünscht, auch einen oder mehrere weitere Wirkstoffe enthalten, z.B. ein oder mehrere Vitamine, Diuretika, Antiphlogistika.

Die erfindungsgemäßen Substanzen werden in der Regel in Analogie zu anderen bekannten, im Handel befindlichen Präparaten, insbesondere aber in Analogie zu den in der EP 0 530 702 A1 beschriebenen Verbindungen verabreicht, vorzugsweise in Dosierungen zwischen etwa 1 mg und 1 g, insbesondere zwischen 10 und 200 mg pro Dosierungseinheit. Die tägliche

Dosierung liegt vorzugsweise zwischen etwa 0,1 und 50 mg/kg, insbesondere 1 und 10 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und/oder durch Kristallisation. Rf-Werte an Kieselgel, Laufmittel Petrolether/Methyl-tert.-butylether 1:1, wenn nicht anders angegeben. FAB = massenspektrometrisch nach der "Fast atom bombardment"-Methode erhaltener Molionen-Peak (M⁺+1).

### Beispiel 1

Eine Lösung von 2,72 g 1,2-Dthydro-2-oxo-3-N-methyl-N-methylsulfonylaminomethyl-6-butyl-pyridin ("IIIa"; FAB 273; erhältlich durch Reaktion von 1,2-Dihydro-2-oxo-6-butyl-pyridin-3-carboxaldehyd mit Methylamin/H₂ zu 1,2-Dihydro-2-oxo-3-methylaminomethyl-6-butyl-pyridin und Umsetzung mit Methansulfonylchlorid) in 25 ml DMF wird mit 1,12 g K-tert.-Butylat versetzt. Man rührt 10 Minuten bei 20°, tropft innerhalb 30 Minuten eine Lösung von 2,72 g 4'-Brommethyl-2-cyan-biphenyl ("IIa") in 15 ml DMF hinzu und rührt 16 Std. bei 20°. Nach dem Eindampfen arbeitet man wie üblich auf und erhält 1,2-Dihydro-1-(2'-cyanbiphenylyl-4-methyl)-2-oxo-3-N-methyl-N-methylsulfonyl-aminomethyl-6-butyl-pyridin ("Ix"), Öl, Rf 0,35 (Petrolether/Ethylacetat 4:6).

Analog erhält man aus 1,2-Dihydro-2-oxo-3-N-isopropyl-N-methylsulfonylaminomethyl-6-butyl-pyridin (FAB 301) mit "IIa" das 1,2-Dihydro-1-(2'-cyan-biphenylyl-4-methyl)-2-oxo-3-N-isopropyl-N-methylsulfonyl-aminomethyl)-6-butyl-pyridin, F. 155°.

### Beispiel 2

Eine Suspension von 1,2 g 1,2-Dihydro-1-(2'-(1H(oder 2H)-1(oder 2)-triphenylmethyl-5-tetrazolyl)-biphenylyl-4-methyl)-2-oxo-3-N-methyl-N-methylsulfonyl-aminomethyl-6-butyl-pyridin (erhältlich aus IIIa und 4-Brommethyl-2'-(1H (oder 2H)-1(oder 2)-triphenylmethyl-5-tetrazolyly)-biphenyl in 80 ml Methanol wird mit 20 ml Ameisensäure versetzt und 2 Std. bei 50° gerührt. Man konzentriert die Lösung, chromatographiert den Rückstand an Kieselgel und erhält 1,2-Dihydro-1-(2'-(1H-5-tetrazolyl)-biphenylyl-4-methyl)-2-oxo-3-N-methyl-N-methylsulfonyl-aminomethyl-6-butyl-pyridin ("Iy"), F. 104°. Mit KOH in Ethanol wird daraus das K-Salz hergestellt, F. 169°.

Analog erhält man aus 1,2-Dihydro-1-(2'-(1H(oder 2H)-1(oder 2)-triphenylmethyl-5-tetrazolyl)-biphenylyl-4-methyl)-2-oxo-3-N-isopropyl-N-methylsulfonyl-aminomethyl-6-butyl-pyridin mit Ameisensäure in Methanol das 1,2-Dihydro-1-(2'-(1H-5-tetrazolyl)-biphenylyl-4-methyl)-2-oxo-3-N-isopropyl-N-methylsulfonyl-aminomethyl-6-butyl-pyridin ("Iz"), F. 106°; K-Salz, F. 152°.

### Beispiel 3

Eine Lösung von 4,35 g 1,2-Dihydro-1-(2'-cyanbiphenylyl-4-methyl)-2-oxo-3-methylsulfonylaminomethyl-6-butyl-pyridin (F. 59°; vgl. EP 0 530 702 A1, Beispiel 21) in 16 ml DMF wird mit 10 g Cs₂CO₃ versetzt und 30 Min. gerührt. Man gibt 3 ml Methyliodid hinzu und rührt 16 Std. bei 20°. Nach üblicher Aufarbeitung erhält man "Ix", Rf 0,35.

Analog erhält man mit Ethyliodid, Propyliodid, Isopropyliodid, Butyliodid Isobutyliodid, sek.-Butylbromid, tert.-Butylbromid, N-Pentylbromid oder Cyclopropylmethylbromid die nachstehenden 1,2-Dihydro-1-(2'-cyan-biphenylyl)-4-methyl)-2-oxo-6-butyl-pyridine:
3-N-Ethyl-N-methylsulfonyl-aminomethyl-, Öl, Rf 0,75
3-N-Propyl-N-methylsulfonyl-aminomethyl-, F. 118 °
3-N-[Isopropyl-N-methylsulfonyl-aminomethyl-, F. 155°
3-N-Butyl-N-methylsulfonyl-aminomethyl-, Öl, Rf 0,71
3-N-Isobutyl-N-methylsulfonyl-aminomethyl-, Öl, Rf 0,62
3-N-sek.-Butyl-N-methylsulfonyl-aminomethyl-
3-N-tert.-Butyl-N-methylsulfonyl-aminomethyl-3-N-Pentyl-N-methylsulfonyl-aminomethyl-, Öl, Rf 0,80
3-N-Cyclopropylmethyl-N-methylsulfonyl-aminomethyl-, F. 93 °.

Analog erhält man aus 1,2-Dihydro-1-(2'-cyanbiphenylyl-4-methyl)-2-oxo-3-methylsulfonylaminomethyl-6-propyl-pyridin mit den oben genannten Alkylhalogeniden die nachstehenden 1,2-Dihydro-1-(2'-cyanbiphenylyl-4-methyl)-2-oxo-6-propyl-pyridine:
3-N-Methyl-N-methylsulfonyl-aminomethyl-
3-N-Ethyl-N-methylsulfonylaminomethyl-
3-N-Propyl-N-methylsulfonyl-aminomethyl-
3-N-Isopropyl-N-methylsulfonyl-aminomethyl-
3-N-Butyl-N-methylsulfonyl-aminomethyl-
3-N-Isobutyl-N-methylsulfonyl-aminomethyl-
3-N-sek.-Butyl-N-methylsulfonyl-aminomethyl-
3-N-tert.-Butyl-N-methylsulfonyl-aminomethyl-
3-N-Pentyl-N-methylsulfonyl-aminomethyl-
3-N-Cyclopropylmethyl-N-methylsulfonyl-aminomethyl-.

### Beispiel 4

Ein Gemisch von 463 mg "Ix", 200 mg Trimethylzinnazid und 12 ml Xylol wird 96 Std. gekocht; nach 48 Std. gibt man nochmals 200 mg Trimethylzinnazid hinzu. Man kühlt ab, versetzt mit etherischer Salzsäure und dampft ein. Chromatographie des Rückstands (Kieselgel; Dichlormethan/Methanol 9:1) liefert "Iy", F. 104°.

Analog erhält man aus den in Beispiel 3 angegebenen 2'-Cyanverbindungen mit Trimethylzinnazid in Xylol die nachstehenden 1,2-Dihydro-1-(2'-(1H-5-tetrazolyl)-biphenylyl-4-methyl)-2-oxo-6-butyl-pyridine:
3-N-Ethyl-N-methylsulfonyl-aminomethyl-, F. 109 °; K-Salz, F. 81 °
3-N-Propyl-N-methylsulfonyl-aminomethyl-, F. > 300°; K-Salz, F. 104 °
3-N-Isopropyl-N-methylsulfonyl-aminomethyl- ("Iz"), F.106°
3-N-Butyl-N-methylsulfonyl-aminomethyl-, F. 95 °; K-Salz, F. 93 °
3-N-Isobutyl-N-methylsulfonyl-aminomethyl-, F. 117 °; K-Salz, F. 100 °
3-N-sek.-Butyl-N-methylsulfonyl-aminomethyl-
3-N-tert.-Butyl-N-methylsulfonyl-aminomethyl-
3-N-Pentyl-N-methylsulfonyl-aminomethyl-, F. 63 °; K-Salz, F. 96 °
3-N-Cyclopropylmethyl-N-methylsulfonyl-aminomethyl-, F. 96 °; K-Salz, F. 103 °
sowie die nachstehenden 1,2-Dihydro-1-(2'-(1H-5-tetrazolyl)-biphenylyl-4-methyl)-2-oxo-6-propyl-pyridine:
3-N-Methyl-N-methylsulfonyl-aminomethyl-
3-N-Ethyl-N-methylsulfonyl-aminomethyl-
3-N-Propyl-N-methylsulfonyl-aminomethyl-
3-N-Isopropyl-N-methylsulfonyl-aminomethyl-
3-N-Butyl-N-methylsulfonyl-aminomethyl-
3-N-Isobutyl-N-methylsulfonyl-aminomethyl-
3-N-sek.-Butyl-N-methylsulfonyl-aminomethyl-
3-N-tert.-Butyl-N-methylsulfonyl-aminomethyl-
3-N-Pentyl-N-ethylsulfonyl-aminomethyl-
3-N-Cyclopropylmethy-N-methylsulfonyl-aminomethyl-, Rf 0,65
(Dichlormethan/Methanol 9 : 1); K-Salz, F. 119°.

### Beispiel 5

Ein Gemisch von 463 mg "Ix", 700 mg Triethylammoniumchlorid, 700 mg NaN₃ und 4 ml DMF wird 36 Std. bei 120° gerührt. Man kühlt ab, filtriert das gebildete NaCl ab, dampft ein, arbeitet wie üblich auf (wässerige Salzsäure/Dichlormethan) und erhält "Iy", F. 104°.

Die nachstehenden Beispiele betreffen pharmazeutische Zubereitungen, die Wirkstoffe der Formel I oder ihre Salze enthalten.

### Beispiel A: Tabletten und Dragees

In üblicher Weise werden Tabletten folgender Zusammensetzung gepreßt, die bei Bedarf mit einer üblichen Drageedecke auf Sucrosegrundlage überzogen werden:

| | |
|---|---|
| Wirkstoff der Formel I | 100 mg |
| Mikrokristalline Cellulose | 278,8 mg |
| Lactose | 110 mg |
| Maisstärke | 11 mg |
| Magnesiumstearat | 5 mg |
| Feinteiliges Siliciumdioxid | 0,2 mg |

### Beispiel B: Hartgelatine-Kapseln

Übliche zweiteilige Hartgelatine-Kapseln werden jeweils gefüllt mit

| | |
|---|---|
| Wirkstoff der Formel I | 100 mg |
| Lactose | 150 mg |
| Cellulose | 50 mg |
| Magnesiumstearat | 6 mg |

### Beispiel C: Weichgelatine-Kapseln

Übliche Weichgelatine-Kapseln werden mit einem Gemisch aus jeweils 50 mg Wirkstoff der Formel I und 250 mg Olivenöl gefüllt.

### Beispiel D: Ampullen

Eine Lösung von 200 g Wirkstoff der Formel I in 2 kg 1,2-Propandiol wird mit Wasser auf 10 l aufgefüllt und in Ampullen gefüllt, so daß jede Ampulle 20 mg Wirkstoff enthält.

## Patentansprüche

1. 1,2-Dihydro-2-oxo-3-methylsulfonylaminomethyl-pyridine der Formel I 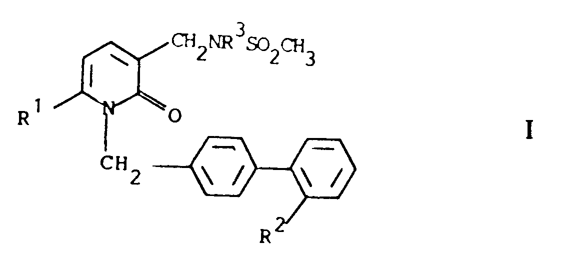 worin
R¹ Alkyl mit 1-5 C-Atomen,
R² CN oder 1H-5-Tetrazolyl und
R³ Alkyl mit 1-5 C-Atomen oder Cyclopropylmethyl bedeuten,
sowie ihre Salze.

2. a) 1,2-Dihydro-1-(2'-(1H-5-tetrazolyl)-biphenylyl-4-methyl)-2-oxo-3-N-methyl-N-methylsulfonyl-aminomethyl-6-butyl-pyridin und dessen Kaliumsalz;
b) 1,2-Dihydro-1-(2'-(1H-5-tetrazolyl)-biphenylyl-4-methyl)-2-oxo-3-N-isopropyl-N-methylsulfonyl-aminomethyl-6-butyl-pyridin und dessen Kaliumsalz;
c) 1,2-Dihydro-1-(2'-(1H-5-tetrazolyl)-biphenylyl-4-methyl)-2-oxo-3-N-cyclopropylmethyl-N-methylsulfonyl-aminomethyl-6-butyl-pyridin und dessen Kaliumsalz.

3. Verfahren zur Herstellung von Verbindungen der Formel I nach Anspruch 1 sowie ihrer Salze, dadurch gekennzeichnet, daß man
a) eine Verbindung der Formel II 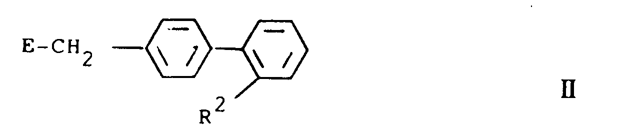 worin
E Cl, Br, I oder eine freie oder reaktionsfähig funktionell abgewandelte OH-Gruppe bedeutet und
R² die bei Formel I angegebene Bedeutung hat,
mit einer Verbindung der Formel III 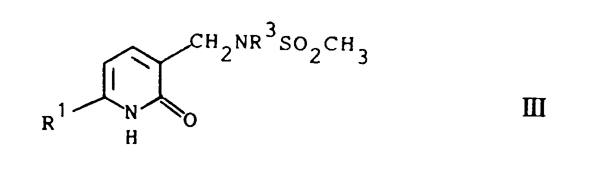 worin
R¹ und R³ die bei Formel I angegebenen Bedeutungen haben, oder einem reäktionsfähigen Derivat dieser Verbindung umsetzt, oder daß man
b) eine Verbindung der Formel I aus einem ihrer funktionellen Derivate durch Behandeln mit einem solvolysierenden oder hydrogenolysierenden Mittel in Freiheit setzt, oder daß man
c) eine Verbindung der Formel IV 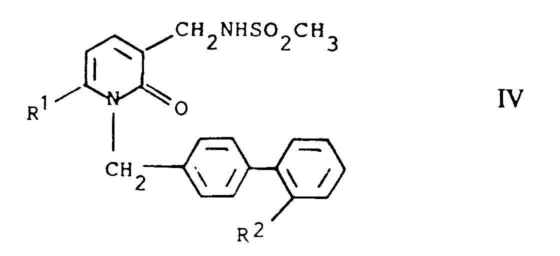 worin
R¹ und R² die bei Formel I angegebenen Bedeutungen haben, oder ein reaktionsfähiges Derivat dieser Verbindung
mit einer Verbindung der Formel V
E-R³ V
worin
E und R³ die bei den Formeln II bzw. I angegebenen Bedeutungen haben,
umsetzt
und/oder daß man in einer Verbindung der Formel I, worin R² CN bedeutet, diesen Rest CN in eine 1H-5-Tetrazolylgruppe umwandelt und/oder eine Verbindung der Formel I, worin R² 1H-5-Tetrazolyl bedeutet, durch Behandeln mit einer Base in eines ihrer Salze umwandelt,

4. Verfahren zur Herstellung pharmazeutischer Zubereitungen, dadurch gekennzeichnet, daß man eine Verbindung der Formel I nach Anspruch 1 und/oder eines ihrer physiologisch unbedenklichen Salze zusammen mit mindestens einem festen, flüssigen oder halbflüssigen Träger- oder Hilfsstoff in eine geeingete Dosierungsform bringt.

5. Pharmazeutische Zubereitung, gekennzeichnet durch einen Gehalt an mindestens einer Verbindung der Formel I nach Anspruch 1 und/oder einem ihrer physiologisch unbedenklichen Salze.

6. Verbindungen der Formel I nach Anspruch 1 und ihrer physiologisch unbedenklichen Salze zur Bekämpfung von Krankheiten.

7. Verwendung von Verbindungen der Formel I nach Anspruch 1 und/oder ihrer physiologisch unbedenklichen Salze zur Herstellung eines Arzneimittels.

8. Verwendung von Verbindungen der Formel I nach Anspruch 1 und/oder ihrer physiologisch unbedenklichen Salze bei der Bekämpfung von Krankheiten.
